# EUROPÄISCHE PATENTSCHRIFT

(11) **EP 1 523 337 B1**
(45) Veröffentlichungstag und Bekanntmachung des Hinweises auf die Patenterteilung: **18.10.2006**
(21) Anmeldenummer: 03757058.7
(22) Anmeldetag: 11.06.2003
(51) Int. Cl.: A61K 49/00, A61K 51/12

(54) **BILDGEBENDES VERFAHREN UND VORRICHTUNG ZU DESSEN DURCHFUHRUNG**
IMAGING METHOD AND DEVICE FOR CARRYING OUT SAID METHOD
PROCEDE D'IMAGERIE ET DISPOSITIF POUR SON EXECUTION

(30) Priorität: 11.06.2002 DE 10225932
(43) Veröffentlichungstag der Anmeldung: 20.04.2005
(73) Patentinhaber: Deutsches Krebsforschungszentrum Stiftung des öffentlichen Rechts, 69120 Heidelberg (DE)
(72) Erfinder: PETER, Jörg, 69121 Heidelberg (DE)
(74) Vertreter: Isenbruck, Günter
(86) Internationale Anmeldenummer: PCT/EP2003/006102
(87) Internationale Veröffentlichungsnummer: WO 2003/104799

(56) Entgegenhaltungen:
- WO-A-01/56582
- HOOPER C E ET AL: "CCD IMAGING OF LUCIFERASE GENE EXPRESSION IN SINGLE MAMMALIAN CELLS" JOURNAL OF BIOLUMINESCENCE AND CHEMILUMINESCENCE, WILEY AND SONS, CHICHESTER, GB, Bd. 5, 1990, Seiten 123-130, XP000186189 ISSN: 0884-3996
- JACOBS A ET AL: "Functional coexpression of HSV-1 thymidine kinase and green fluorescent protein: implications for noninvasive imaging of transgene expression." NEOPLASIA (NEW YORK, N.Y.) JUN 1999, Bd. 1, Nr. 2, Juni 1999 (1999-06), Seiten 154-161, XP0008024072 ISSN: 1522-8002
- KOST BENEDIKT ET AL: "Non-destructive detection of firefly luciferase (LUC) activity in single plant cells using a cooled, slow-scan CCD camera and an optimized assay" PLANT JOURNAL, Bd. 8, Nr. 1, 1995, Seiten 155-166, XP002260828 & ISSN: 0960-7412
- EDINGER MATTHIAS ET AL: "Monitoring the anti tumor activity of expanded CD8+ NKT cells after allogeneic bone marrow transplantation using bioluminescent imaging" BLOOD, Bd. 98, Nr. 11 Part 1, 16. November 2001 (2001-11-16), Seite 433a, XP0008024079 & 43RD ANNUAL MEETING OF THE AMERICAN SOCIETY OF HEMATOLOGY, PART 1; ORLANDO, FLORIDA, USA; DECEMBER 07-11, 2001 ISSN: 0006-4971
- EBMEIER K P ET AL: "Temporal lobe abnormalities in dementia and depression: a study using high resolution single photon emission tomography and magnetic resonance imaging." JOURNAL OF NEUROLOGY, NEUROSURGERY, AND PSYCHIATRY. NOV 1997, Bd. 63, Nr. 5, November 1997 (1997-11), Seiten 597-604, XP0008024089 ISSN: 0022-3050
- HANDLER A M ET AL: "Polyubiquitin-regulated DsRed marker for transgenic insects." BIOTECHNIQUES. OCT 2001, Bd. 31, Nr. 4, Oktober 2001 (2001-10), Seiten 820 , 824-828, XP0008024071 ISSN: 0736-6205
- ROGUS R D ET AL: "Accuracy of a photogrammetry-based patient positioning and monitoring system for radiation therapy." MEDICAL PHYSICS. MAY 1999, Bd. 26, Nr. 5, Mai 1999 (1999-05), Seiten 721-728, XP002260857 ISSN: 0094-2405
- EDINGER M ET AL: "Advancing animal models of neoplasia through in vivo bioluminescence imaging." EUROPEAN JOURNAL OF CANCER (OXFORD, ENGLAND : 1990) NOV 2002, Bd. 38, Nr. 16, November 2002 (2002-11), Seiten 2128-2136, XP002260827 ISSN: 0959-8049
- EDINGER MATTHIAS ET AL: "Revealing lymphoma growth and the efficacy of immune cell therapies using in vivo bioluminescence imaging." BLOOD. 15 JAN 2003, Bd. 101, Nr. 2, 15. Januar 2003 (2003-01-15), Seiten 640-648, XP002260829 ISSN: 0006-4971

## Beschreibung

Die Erfindung betrifft ein bildgebendes Verfahren zur Bestimmung von in vivo Verteilungen biolumineszenter, fluoreszenter und radioaktiver Marker. Ferner bezieht sie sich auf eine Vorrichtung zur Durchführung des bildgebenden Verfahrens.

Die qualitative und quantitative Erfassung von morphologischen, funktionellen und biochemischen Parametern unter Benutzung bildgebender Verfahren ist Grundlage diverser medizinischer Forschungs- und Anwendungsgebiete. Bekannte, unter anderem in der Tumorforschung eingesetzte, bildgebende Verfahren sind zum Beispiel die Einzelphotonenemissions-Computertomographie (SPECT) unter Einsatz von Radionukliden oder optische Technologien unter Verwendung von Fluoreszenz oder Biolumineszenz.

Bei der Einzelphotonenemissions-Computertomographie werden dem zu untersuchenden Tier oder Menschen radioaktive Marker injiziert, die sich in Abhängigkeit von den sie transportierenden biologischen Trägern in bestimmten Organen oder Gewebearten des Tieres/Menschen konzentrieren. Die radioaktiven Marker geben radioaktive Strahlung (y-Strahlung) ab, deren Intensität in einem bestimmten Bereich des zu untersuchenden Subjekts von der Konzentration des Markers in diesem Bereich abhängt. Die radioaktive Strahlung wird mittels einer y-Kamera oder Szintillations-Kamera detektiert. Zur Durchführung hochauflösender Studien an kleinen Labortieren sind zum Beispiel aus D.P. McElroy et al.: Evaluation of A-SPECT: A Desktop Pinhole SPECT System for Small Animal Imaging, Proc. Med. Imag. Conf. San Diego 2001, M10-4 oder aus A.G. Weisenberger et al.: Development of a Novel Radiation Imaging Detector System for In Vivo Gene Imaging in Small Animal Studies, IEEE Transactions on Nuclear Science, Vol. 45, No. 3 (Juni 1998) 1743-1749 Vorrichtungen zum Nachweis der γ-Photonen bekannt. Anwendungsgebiete solcher Tomographen sind zum Beispiel die präklinische Forschung oder die Radiotracer-Entwicklung.

Ein weiteres im Stand der Technik bekanntes bildgebendes Verfahren zur in vivo Untersuchung ist ein optisches Verfahren, das sich fluoreszenter oder biolumineszenter Marker bedient. Diese dienen beispielsweise als Reportergene, die zur Beobachtung von Gen-Expression eingesetzt werden, da die zugehörigen Proteine ein meßbares optisches Signal produzieren. Das Gen, das das Protein Luziferase codiert, ist ein vielverwendetes Reportergen. Dabei wird das Gen für ein bestimmtes Protein durch das Luziferasegen ersetzt. Bei Aktivierung des zugehörigen Promotors treibt dieser nun die Transkription des Luziferasegens. Das Enzym Luziferase aus dem nordamerikanischen Leuchtkäfer Photinus pyralis katalysiert in Gegenwart von ATP und Mg²⁺ die oxidative Decarboxylierung von Luziferin. Dabei entstehen Lichtblitze, die sich zum Leuchten dieser Tiere aufaddieren. Die bei Anwesenheit von Luziferin und ATP auftretende Lumineszenz zeigt folglich die Expression von Luziferase an. Dieses optische Signal ist leicht meßbar, zum Beispiel mit CCD-Kameras. Ebenso wie biolumineszente Reportergene werden auch fluoreszente Reportergene eingesetzt, beispielsweise das Gen zum grünfluoreszierenden Protein (GFP). Solche Proteine werden durch Bestrahlung mit einer äußeren Lichtquelle zum Fluoreszieren angeregt. In vivo Bildgebung von Genexpression mit optischen Verfahren ist beispielsweise aus C. Bremer, R. Weissleder: In Vivo Imaging of Gene Expression: MR and Optical Technologies, Academic Radiology, Vol. 8, No 1 (Januar 2001) 15-23 bekannt. Diese Verfahren werden unter anderem auch zur in vivo Tumorüberwachung eingesetzt (R. Weissleder et al.: In Vivo Imaging of Tumors with Protease-Activated Near-Infrared Fluorescent Probes, Nature Biotechnology, Vol. 17 (April 1999) 375-378).

Vorteile der die optischen (Fluoreszenz oder Lumineszenz) Photonen emittierenden Moleküle sind u.a., daß sie bestimmte chemische Eigenschaften von medizinischem Interesse besitzen, die den radioaktiv markierten Komponenten fehlen, beispielsweise, daß sie durch spezifische enzymatische Wechselwirkungen aktiviert werden können. Die radioaktiven Substanzen haben hingegen den Vorteil, daß die von ihnen emittierten γ-Strahlen viel geringere Wechselwirkungswahrscheinlichkeiten mit dichtem Gewebe als die optischen Photonen aufweisen, so daß sie große Gewebevolumen oder -dicken durchdringen können. Des weiteren kann ihre geringe oder nicht vorhandene Wechselwirkung mit den biochemischen Eigenschaften des zu untersuchenden Gewebes von Vorteil sein. Im Stand der Technik werden beide bildgebende Verfahren, das Verfahren zum Nachweis der durch die fluoreszierenden oder lumineszierenden Moleküle emittierten optischen Photonen einerseits und das Verfahren zum Nachweis der durch die Radioisotope emittierten höherenergetischen Photonen andererseits, separat, in verschiedenen Vorrichtungen durchgeführt. Ein Vergleich der mit beiden Bildgebungsverfahren gewonnenen Bilder ist nur bedingt möglich, da sie nicht gleichzeitig und unter den gleichen Projektionswinkeln gewonnen werden können. Es ergeben sich die Probleme der übermäßigen Belastung des zu untersuchenden Subjekts, der Nichtreproduzierbarkeit kinetischer Studien, der nicht-identischen Bildgebungsgeometrien, der Tier- und Organbewegung und der korrekten Überlagerung der Bilder, wenn beide Verfahren nacheinander durchgeführt werden.

Der vorliegenden Erfindung liegt daher die Aufgabe zugrunde, die Nachteile des Standes der Technik zu vermeiden und die Vorteile beider oben beschriebener Technologien zu vereinen.

Diese Aufgabe wird durch ein bildgebendes Verfahren zur gleichzeitigen Bestimmung von in vivo Verteilungen bialumineszenter und/oder fluoreszenter Marker und radioaktiver Marker unter gleichen Projektionswinkeln gelöst, wobei die Verteilung der biolumineszenten und/oder fluoreszenten Marker durch separaten Nachweis von Photonen mit einer ersten mittleren Energie, die die biolumineszenten und/oder fluoreszenten Marker emittieren, mittels mindestens einem ersten Detektor bestimmt wird und die Verteilung der radioaktiven Marker durch gleichzeitigen separaten Nachweis von Photonen mit einer zweiten mittleren Energie, die die radioaktiven Marker emittieren, mittels mindestens einem zweiten Detektor bestimmt wird.

Unter Projektion ist in diesem Zusammenhang die zweidimensionale Abbildung einer dreidimensionalen Energieverteilung bei einem bestimmten Projektionsraumwinkel des Detektors gegenüber dem abzubildenden Objekt zu verstehen. In der Erfindung sind die Projektionsraumwinkel der beiden Detektorsysteme gegenüber dem Objekt identisch, d.h. das Objekt wird von einem gleichen Projektionswinkel aus von beiden Detektoren "betrachtet".

Optische Photonen der biolumineszenten und/oder fluoreszenten Marker besitzen eine (erste) mittlere Energie im Bereich zwischen 1eV und 3eV. Photonen der radioaktiven Marker besitzen eine zweite mittlere Energie in einem Bereich zwischen 10keV und 600keV. Das erfindungsgemäße bildgebende Verfahren wird in vivo durchgeführt. Es kann zum Beispiel an lebenden Labortieren angewendet werden. Dabei können in vorteilhafter Weise
- Transport, Metabolismus und Ausscheidung von Wirkstoffen im lebenden Organismus beobachtet und
- biologische Prozesse in ihrer natürlichen Umgebung gemessen werden.

In einer bevorzugten Ausführungsform der vorliegenden Erfindung werden die Photonen der biolumineszenten und/oder fluoreszenten Marker mit der ersten mittleren Energie und die Photonen der radioaktiven Marker mit der zweiten mittleren Energie zum separaten Nachweis mit Hilfe einer Schicht getrennt, wobei die Schicht die Photonen in Abhängigkeit von ihrer Energie im wesentlichen reflektiert oder transmittiert. Beispielsweise wird γ-Strahlung ohne oder mit nur geringen Wechselwirkungen mit der Schicht transmittiert, während die niederenergetischere optische Strahlung durch die Schicht reflektiert wird. Dies ermöglicht einen separaten Nachweis der Photonen verschiedener Energien, die unter demselben Projektionswinkel von Markern in dem zu untersuchenden Subjekt emittiert werden.

In einer bevorzugten Ausführungsform der vorliegenden Erfindung dient die Schicht dazu, die Photonen der biolumineszenten und/oder fluoreszenten Marker in Richtung des mindestens einen ersten Detektors zu reflektieren und die Photonen der radioaktiven Marker in Richtung des mindestens einen zweiten Detektors zu transmittieren. Die Photonen verschiedener Energien werden folglich nicht nur durch die Schicht getrennt, sondern auch schon in Richtung der sie nachweisenden Detektoren "gelenkt".

Vorzugsweise umfassen die biolumineszenten und/oder fluoreszenten Marker mindestens einen Marker aus der Gruppe Marker des Luziferase-Reporters, Marker-Moleküle mit Emissionswellenlängen im Nahinfrarotbereich (NIRF-Moleküle) und Moleküle des GFP (des grünfluoreszierenden Proteins). Marker aus dieser Gruppe wurden bereits erfolgreich in das Reportergenkonzept integriert und in vivo (in lebenden) Tieren nachgewiesen. Die Luziferase produziert im Rahmen einer enzymatischen Reaktion (siehe oben) ein blaues oder gelb-grünes Licht. Die Enzymsubstrate, die die Ausgangssubstanzen für die lichtemittierenden Produkte bilden, heißen Luziferine. Luziferase/Luziferin-Systeme findet man zum Beispiel in der Feuerfliege Photinus pyralis (Emissionsmaximum bei 562 nm), in Leuchtkäfern und in zahlreichen marinen Leuchtbakterien (Emissionsmaximum bei 489 nm).

Die am besten charakterisierten grünfluoreszierenden Proteine (GFP) stammen von der pazifischen Qualle Aequorea victoria und der Seefeder Renilla reniformis. In beiden Fällen transformiert das GFP blaue Chemilumineszenz in grüne Fluoreszenz (Emissionsmaximum bei 508 nm). GFP ist ein relativ kleines Protein, bestehend aus 238 Aminosäuren.

Die Moleküle mit Emissionswellenlängen im Nahinfrarotbereich (NIRF-Marker) haben geringere Wechselwirkungswahrscheinlichkeiten im Gewebe als Photonen mit Wellenlängen im sichtbaren Wellenlängenspektrum und deshalb eine größere Eindringtiefe.

Mit den biolumineszenten und/oder fluoreszenten oder mit den radioaktiven Markern können beispielsweise Proteine, Lipide, RNA oder DNA markiert werden.

Vorzugsweise umfassen die radioaktiven Marker mindestens einen Marker aus der Gruppe As-72, Br-75, Co-55, Cu-61, Cu-64, Cu-67, Ga-67, Gd-153, I-123, I-125, I-131, In-111, Ru-97, T1-201, Tc-99m und Xe-133. Das jeweils verwendete Radioisotop wird in Bezug auf seine Halbwertszeit und die Energie der von ihm emittierten Strahlung in Abhängigkeit von dem zu messenden biologischen Prozeß als Marker ausgewählt.

In einer bevorzugten Ausführungsform der vorliegenden Erfindung wird der Nachweis der die erste mittlere Energie aufweisenden Photonen mit mindestens einer CCD-Kamera und der Nachweis der die zweite mittlere Energie aufweisenden Photonen mit mindestens einem Einzelphotonenemissions-Computertomographie (SPECT)-Detektor, der einen Kollimator mit mindestens einer Apertur umfaßt, durchgeführt.

CCD's (Charge Coupled Device) sind ladungsgekoppelte Abbildungssensoren, die zum hochempfindlichen Photonennachweis dienen. Die CCD-Kamera ist in eine Vielzahl kleiner lichtempfindlicher Zonen (Pixel) aufgeteilt, die die einzelnen Punkte eines Bildes ergeben. Das Raster von Pixels wird durch eine Schaltungsstruktur auf einem Halbleiterkristall (üblicherweise Silicium) gebildet. Die Arbeitsweise der CCD-Kamera basiert auf der Freisetzung von Elektronen durch auftreffendes Licht in dem Halbleitermaterial. Durch ein Photon, das auf ein Pixel fällt, wird mindestens ein Elektron freigesetzt, das durch ein elektrisches Potential am Ort des Pixels festgehalten wird. Die Anzahl der Elektronen, die am Ort des Pixels freigesetzt werden, ist proportional zur Intensität des an diesem Ort einfallenden Lichts. Die Anzahl der Elektronen wird in jedem Pixel gemessen, so daß ein Bild rekonstruiert werden kann. CCD's sollten gekühlt werden, da ansonsten mehr Elektronen ausgelesen würden, die nicht durch den Lichteinfall, sondern durch die Erwärmung freigesetzt würden. Bei der vorliegenden Erfindung werden vorzugsweise die optischen Photonen der biolumineszenten und/oder fluoreszenten Marker mit Hilfe mindestens einer CCD-Kamera detektiert.

SPECT-Detektoren enthalten üblicherweise γ- oder Szintillationskameras. Ein Szintillator absorbiert die von den Radioisotopen emittierten γ-Strahlen. Als Antwort darauf emittiert der Szintillator Lichtszintillationen aus sichtbarem Licht, die durch eine Gruppe von Photomultiplikatoren des SPECT-Detektors erfaßt und in ein meßbares elektrisches Signal umgesetzt werden. Eine Lokalisierung des Ortes der radioaktiven Emission von Photonen in einem Körper kann nur dann erfolgen, wenn zwischen dem Körper und dem Szintillator ein Kollimator angeordnet wird. Dieser Kollimator dient zur Abschirmung von Photonen, welche sich nicht in einem durch die Kollimatorgeometrie definierten Aktzeptanzbereich befinden, von dem Szintillator. Ferner definiert der Kollimator das Gesichtsfeld des Detektorsystems.

Gegenstand der vorliegenden Erfindung ist weiterhin eine Vorrichtung zur Durchführung des erfindungsgemäßen bildgebenden Verfahrens. Diese Vorrichtung enthält mindestens eine gekühlte CCD-Kamera als ersten Detektor, mindestens einen Einzelphotonenemissions-Computertomographie (SPECT)-Detektor als zweiten Detektor und eine Schicht, die die Photonen der biolumineszenten und/oder fluoreszenten Marker im wesentlichen reflektiert und die Photonen der radioaktiven Marker im wesentlichen transmittiert. Die Schicht dient (wie oben schon erwähnt) zur Trennung der Photonen verschiedener Energien, die in verschiedenen Detektoren (CCD-Kamera und SPECT-Detektor) detektiert werden. Der SPECT-Detektor umfaßt vorzugsweise einen Kollimator, einen Szintillator und eine Vielzahl von Photomultiplikatoren mit zugehörigen elektronischen Elementen.

Die Detektoren und die Schicht sind bei der vorliegenden Erfindung vorzugsweise in einer vorgegebenen räumlichen Anordnung in einem gemeinsamen Gehäuse fest installiert. Da CCD's unempfindlich gegenüber dem Energiespektrum der von den Radionukliden emittierten Photonen sind, ist keine Abschirmung notwendig und die CCD's können vollständig in den insgesamt abgeschirmten Tomographen integriert werden.

Je nach Anordnung der Detektoren wird bei der vorliegenden Erfindung eine hochreflektierende oder eine diffus reflektierende Schicht eingesetzt. Als hochreflektierende Schicht wird beispielsweise Aluminium auf ein geeignetes Basismaterial mit geringem Schwächungskoeffizient aufgedampft. Solche Schichten sind im Stand der Technik verfügbar. Ebenso sind diffus reflektierende dünne Schichten im Stand der Technik erhältlich, beispielsweise in Form von auf ein geeignetes Basismaterial aufgebrachten Mikrokörnern aus Kunststoff. Die Schichten sollen bei der vorliegenden Erfindung so dünn wie möglich sein, um eine minimale Schwächung und Streuung der radioisotopischen Photonen zu gewährleisten, so dass diese Effekte prinzipiell vernachlässigbar sind. Wenn überhaupt vorhanden, können Streuung und Absorption durch die Schicht in einer der Detektion folgenden Bildrekonstruktion kompensiert werden. Die mininimale Dicke wird durch die notwendigen statischen Eigenschaften, beispielsweise die planare Steifigkeit, bestimmt

In einer bevorzugten Ausführungsform der vorliegenden Erfindung umfaßt der mindestens eine SPECT-Detektor ein (z.B. planares) Szintillations-Kristallfeld mit einer Vielzahl von Szintillations-Kristallen und ein ortsauflösendes Photomultiplikatorfeld. Die Szintillationskristalle sind dichte, transparente kristalline Materialien (zum Beispiel NaI(Tl)), die als Umwandler für hochenergetische γ-Strahlen in sichtbares Licht dienen. Das sichtbare Licht wird von dem Photomultiplikatorfeld ortsauflösend in Form elektrischer Signale detektiert.

Gegenstand der vorliegenden Erfindung ist weiterhin ein bildgebendes Verfahren zur abwechselnden Bestimmung von in vivo Verteilungen biolumineszenter und/oder fluoreszenter Marker und von in vivo Verteilungen radioaktiver Marker mit einem gemeinsamen Messaufbau unter gleichen Projektionswinkeln, wobei die Verteilung der biolumineszenten und/oder fluoreszenten Marker durch separaten Nachweis von Photonen mit einer ersten mittleren Energie, die die biolumineszenten und/oder fluoreszenten Marker emittieren, mittels mindestens einem ersten Detektor bestimmt wird und abwechselnd dazu die Verteilung der radioaktiven Marker durch separaten Nachweis von Photonen mit einer zweiten mittleren Energie, die die radioaktiven Marker emittieren, mittels mindestens einem zweiten Detektor bestimmt wird.

Zur Durchführung dieses erfindungsgemäßen Verfahrens dient vorzugsweise eine Vorrichtung, bei der die für die SPECT-Detektoren als Kollimatoren dienenden Masken während einer Messung aus den Gesichtsfeldern der CCD-Kameras herausfahrbar und wieder in die Gesichtsfelder hereinfahrbar sind.

Befinden sich die Masken dabei außerhalb der Gesichtsfelder der CCD-Kameras, so erhöht sich signifikant die Sensitivität dieser optischen bildgebenden Systeme. Die Detektion von radioistopischen Photonen ist in diesem Zustand (ohne Kollimation) jedoch nicht möglich. Daher sind die SPECT-Detektoren bei aus dem Strahlengang entfernten Masken vorzugsweise inaktiv. Befinden sich die Masken in dem Strahlengang, so werden bevorzugt die CCD-Kameras inaktiviert. Durch zeitlich alternierende Einführung der Masken in die beiden Positionen (innerhalb/außerhalb des Gesichtsfeldes der CCD-Kameras) ergibt sich ein bevorzugter Anwendungsmodus der erfindungsgemäßen Vorrichtung.

Die vorliegende Erfindung wird vorzugsweise für in vivo Studien an kleinen Tieren (zum Beispiel Mäusen oder Ratten), zur in vivo Beobachtung von Genexpression und zur Brust-, Prostata-, Hauttumoren- und Schilddrüsenbildgebung verwendet.

Anhand der Zeichnung wird die vorliegende Erfindung nachstehend näher erläutert.

Es zeigt:
- Figur 1: eine bevorzugte Ausführungsform einer erfindungsgemäßen Vorrichtung zur Durchführung des erfindungsgemäßen bildgebenen Verfahrens,
- Figur 2: eine schematische Darstellung der räumlichen Anordnung der Detektoren bei einer weiteren möglichen Ausführungsform der erfindungsgemäßen Vorrichtung,
- Figur 3: eine weitere bevorzugte Ausffihrungsform einer erfindungsgemäßen Vorrichtung mit zwei CCD-Kameras und zwei SPECT-Detektoren,
- Figur 4: eine weitere Ausführungsform einer erfindungsgemäßen Vorrichtung mit zwei CCD-Kameras und zwei SPECT-Detektoren und
- Figur 5: eine Abwandlung der in Figur 3 gezeigten Ausführungsform einer erfindungsgemäßen Vorrichtung mit verfahrbaren Masken und
- Figur 6: eine bevorzugte Abwandlung der in Figur 1 gezeigten Ausführungsform einer erfindungsgemäßen Vorrichtung mit lediglich einer CCD-Kamera

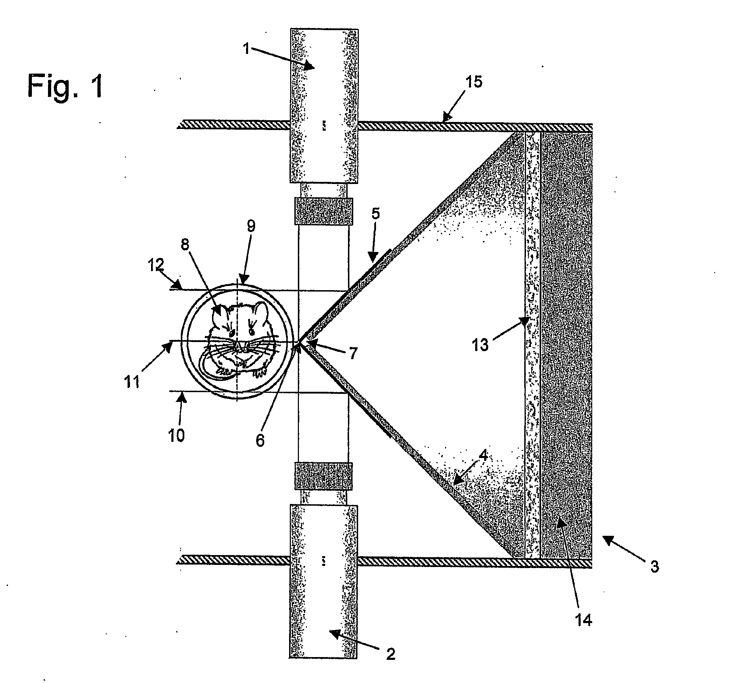

Figur 1 zeigt eine bevorzugte Ausführungsform einer erfindungsgemäßen Vorrichtung mit zwei CCD-Kameras und einem SPECT-Detektor.

Bei dieser bevorzugten Ausführungsform der vorliegenden Erfindung umfaßt die erfindungsgemäße Vorrichtung zwei einander zugewandte CCD-Kameras 1, 2, einen senkrecht zu den CCD-Kameras 1, 2 angeordneten SPECT-Detektor 3, eine vor dem SPECT-Detektor 3 angeordnete, in einem 90°-Winkel gebogene Abschirmung 4 und eine auf der Abschirmung 4 befestigte, ebenfalls in einem 90°-Winkel gebogene Schicht 5, deren Biegekante 6 auf der Biegekante der Abschirmung 4 liegt, wobei die Schicht 5 eine Apertur 7 in der Abschirmung 4 überdeckt. Die Schicht 5 reflektiert optische Photonen, beispielsweise Photonen von biolumineszenten und/oder fluoreszenten Markern, und transmittiert γ-Photonen, beispielsweise Photonen von radioaktiven Markern. Die Abschirmung 4 mit der Apertur 7 dient als Kollimator für den SPECT-Detektor 3. Möglichst nah vor diesem Kollimator wird das zu untersuchende Subjekt angeordnet (hier die Maus 8), das sich vorzugsweise in einer dünnwandigen transparenten Röhre 9 aus Plexiglas befindet. Wenn sich in der Maus 8 radioaktive und biolumineszente und/oder fluoreszente Marker befinden und wenn diese Marker Photonen in Richtung der Abschirmung 4 emittieren, so werden die Photonen in Abhängigkeit von ihrer Energie an der Schicht 5 weitgehend reflektiert oder von der Schicht 5 weitgehend transmittiert. Für niederenergetische, optische Strahlung sind in Figur 1 drei Strahlenverläufe 10, 11, 12 eingezeichnet. Die drei Strahlen 10, 11, 12 werden an der Schicht 5 in einem 90°-Winkel reflektiert, so daß sie direkt zu den CCD-Kameras 1, 2 gelenkt und dort detektiert werden. Die mittel- oder hochenergetischen γ-Photonen können die Schicht 5 ohne oder nur mit geringen Wechselwirkungen passieren. Dann werden sie entweder durch die Abschirmung (zum Beispiel aus Blei oder Wolfram) absorbiert oder sie gelangen durch die Apertur 7 in den SPECT-Detektor 3, wo sie detektiert werden. Der SPECT-Detektor 3 umfaßt ein Szintillationskristallfeld 13 und ein Photomultiplikatorfeld 14, die die einfallenden γ-Photonen in optische Photonen und anschließend in einen elektrischen Strom transformieren. Der SPECT-Detektor 3 mit der Abschirmung 4 und der Schicht 5 und die CCD-Kameras 1, 2 sind fest in einem Gehäuse 15 angeordnet, so daß sie relativ zueinander eine bestimmte räumliche Anordnung beibehalten. Das Gehäuse 15 mit den fest angeordneten Elementen ist um die Röhre 9 mit der Maus 8, vorzugsweise um 360°, rotierbar, zur Gewinnung von Meßdaten unter verschiedenen Winkeln. Denkbar ist hingegen auch eine Rotation der Röhre 9 mitsamt der Maus 8 in dem fest angeordneten Gehäuse 15.

Der Projektionsraumwinkel in Fig.1 beträgt laut Definition 0 Grad. Die Abbildungsebenen der Detektoren sind parallel zueinander (d. h. haben den gleichen Projektionswinkel von 0 Grad), obwohl die Abbildungsebenen der CCD-Kameras 1, 2 um jeweils plus/minus 90 Grad gegenüber der Abbildungsebene des SPECT-Systems 3 rotiert sind. Identische Projektionswinkel sind deshalb gegeben, da auch die Gesichtsfelder (Photonenprojektionstrajektorien) beider CCD's 1, 2 eine Rotation um 90 Grad erfahren (durch die reflektierende Schicht 5).

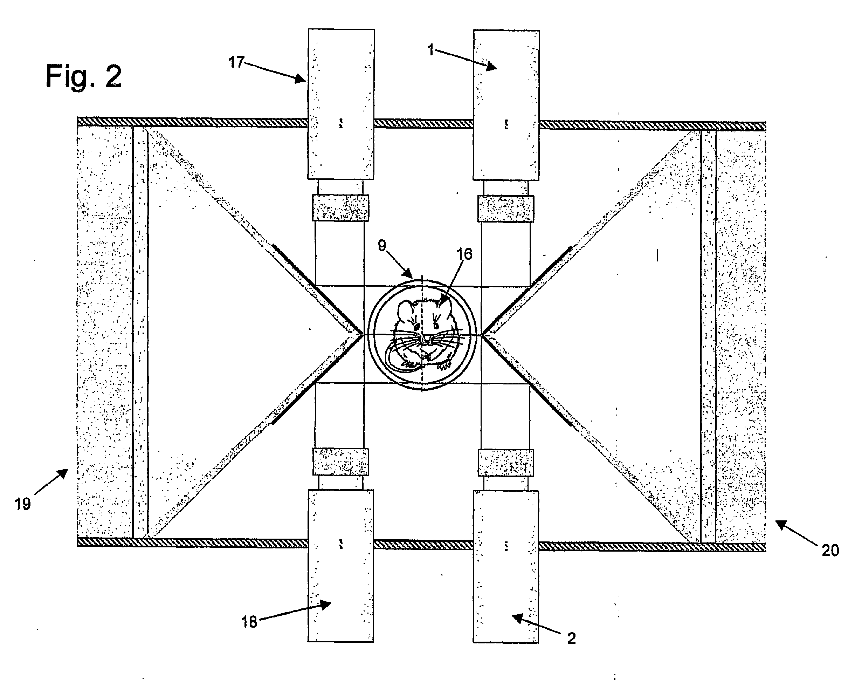

Figur 2 zeigt eine schematische Darstellung der räumlichen Anordnung der Detektoren bei einer weiteren möglichen Ausführungsform der vorliegenden Erfindung.

Dabei wurde das Design aus Figur 1 übernommen und an einer parallel zu der CCD-Kamera-Längsachse mittig durch das in eine Röhre 9 plazierte Subjekt 16 verlaufenden axialen Rotationsachse gespiegelt noch zusätzlich aufgebaut. So können nach der einen Richtung emittierte Photonen durch eine erste und eine zweite CCD-Kamera 1, 2 und einen ersten SPECT-Detektor 20 und in der entgegengesetzten Richtung emittierte Photonen durch eine dritte und vierte CCD-Kamera 17, 18 und einen zweiten SPECT-Detektor 19 detektiert werden.

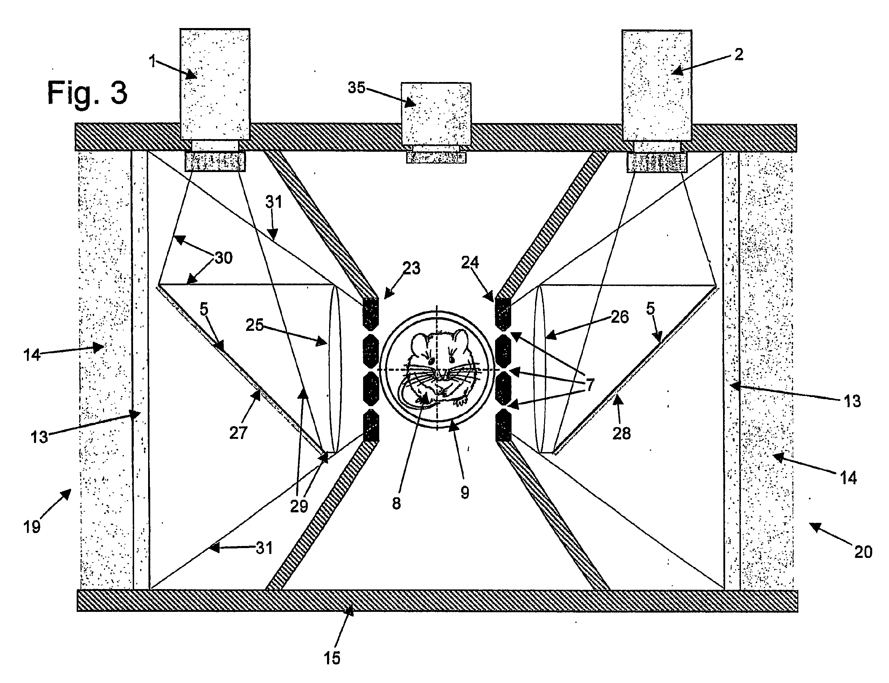

Figur 3 zeigt eine weitere bevorzugte Ausführungsform einer erfindungsgemäßen Vorrichtung mit zwei CCD-Kameras und zwei SPECT-Detektoren.

Bei dieser bevorzugten Ausführungsform der vorliegenden Erfindung umfaßt die erfindungsgemäße Vorrichtung zwei in gleicher Richtung ausgerichtete und voneinander beabstandete CCD-Kameras 1, 2, zwei senkrecht zu den CCD-Kameras 1, 2 angeordnete SPECT-Detektoren 19, 20, zwei Masken 23, 24 mit mindestens je zwei Aperturen 7 und zwei Linsen 25, 26 zwischen den zwei SPECT-Detektoren 19, 20. Ferner enthält die Vorrichtung zwei im wesentlichen je eine Schicht 5 umfassende Reflektoren 27, 28, die so ausgerichtet sind, daß sie die von den biolumineszenten und/oder fluoreszenten Markern emittierten, durch die Aperturen in den Masken in Richtung SPECT-Detektoren durchgelassenen und mit den Linsen fokussierten Photonen weitgehend in Richtung der CCD-Kameras reflektieren. Die von den in der Maus 8 enthaltenen biolumineszenten und/oder fluoreszenten und radioaktiven Marker emittierten Photonen verschiedener Energie müssen zunächst die als SPECT Kollimatoren dienenden Masken 23, 24 mit den Aperturen 7 passieren. Anschließend werden die optischen Photonen durch die Linsen 25, 26 auf die Schicht 5 des jeweiligen Reflektors 27, 28 fokussiert, an der hochreflektierenden Schicht 5 in Richtung der jeweiligen CCD-Kamera 1, 2 reflektiert und dort detektiert. Der Strahlenverlauf zweier optischer Strahlen 29, 30 ist in Figur 3 eingezeichnet. Die γ-Photonen wechselwirken gar nicht oder kaum mit den Linsen 25, 26 und den Reflektoren 27, 28, so daß sie sich in dem Akzeptanz-Kegel 31 der Aperturen 7 ungehindert in Richtung Szintillationskristallfeld 13 des jeweiligen SPECT-Detektors 19, 20, in dem sie detektiert werden, ausbreiten können. Durch die Linsen 25, 26 und Schichten 5 verursachte Streuung und Absorption (soweit vorhanden) können in einer der Akquisition folgenden mathematischen Bildrekonstruktion kompensiert werden. Die Linsen 25, 26 bestehen vorzugsweise aus Materialien, die einen geringen Schwächungskoeffizienten für die radioisotopischen Photonen aufweisen, beispielsweise Plexiglas.

Die gesamte in dem Gehäuse 15 zusammengefaßte starre Anordnung (CCD-Kameras 1, 2, Reflektoren 27, 28, Linsen 25, 26, Masken 23, 24 und SPECT-Detektoren 19, 20) ist, vorzugsweise um 360°, um die Röhre 9 rotierbar, um eine Serie von Meßdaten in gleichen Winkelabständen rund um das zu untersuchende Subjekt erhalten zu können.

Zur Untersuchung der Maus 8 genügt grundsätzlich auch nur die halbe in Figur 3 dargestellte Vorrichtung, also eine CCD-Kamera 1, eine Maske 23, ein SPECT-Detektor 19, eine Linse 25 und ein Reflektor 27 mit einer Schicht 5. Die in Figur 3 gezeigte doppelte Anordnung hat jedoch den Vorteil, daß bei tomographischer Anwendung des Designs die Sensitivität des Kamerasystems bei gleicher Akquisitionszeit doppelt so hoch ist

Optional umfaßt die in Figur 3 dargestellte erfindungsgemäße Vorrichtung einen Positionssensor 35 zur Bestimmung der aktuellen Position der Maus, die sich während einer Messung gegebenenfalls in der Röhre 9 bewegt. Dabei handelt es sich um ein optisches (Standard-) Positioniersystem, welches kontinuierlich die Position von extern an die Maus angebrachten Markern aufzeichnet. Dieses zusätzliche Positioniersystem ist nur dann notwendig, wenn eine Bewegung der Maus in der Röhre 9 zugelassen wird, da dann eine exakte Ableitung (und Kompensation) der Bewegung des Tieres aus den in vivo akquirierten Verteilungen nicht möglich ist.

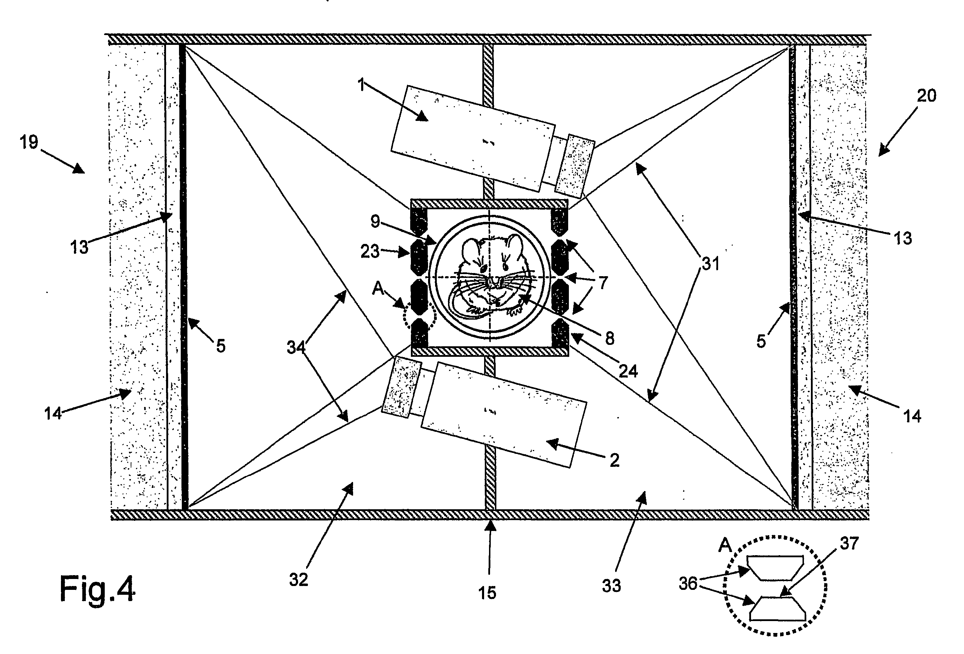

Figur 4 zeigt eine weitere Ausführungsform einer erfindungsgemäßen Vorrichtung.

Bei dieser bevorzugten Ausführungsform der vorliegenden Erfindung umfaßt die erfindungsgemäße Vorrichtung zwei parallel und entgegengesetzt zueinander ausgerichtete CCD-Kameras 1, 2 zwischen zwei einander zugewandten SPECT-Detektoren 19, 20 und zwei Masken 23, 24 mit je mindestens zwei Aperturen 7, wobei sich je eine Schicht 5 vor den SPECT-Detektoren 19, 20 befindet, die die von den biolumineszenten und/oder fluoreszenten Markern emittierten Photonen weitgehend reflektiert und die von den radioaktiven Markern emittierten Photonen weitgehend transmittiert. Das Gehäuse 15 ist dabei in zwei symmetrisch aufgebaute Kammern 32, 33 unterteilt, in deren Mitte sich das zu untersuchende Subjekt (Maus 8) in einer Röhre 9 befindet, um das die gesamte in dem Gehäuse 15 zusammengefaßte Anordnung drehbar gelagert ist, vorzugsweise um 360°. Auf gegenüberliegenden Seiten der Maus 8 befinden sich die Masken 23, 24, durch deren Aperturen 7 ein Teil der von den fluoreszenten, lumineszenten und radioaktiven Markern emittierten Photonen in den Akzeptanz-Kegel 31 der SPECT-Detektoren gelangt. Die Photonen der radioaktiven Marker gehen weitgehend ohne Wechselwirkung durch die Schicht 5 und werden anschließend durch den SPECT-Detektor 19, 20 detektiert. Die Photonen geringerer Energie von den fluoreszenten oder biolumineszenten Markern werden an der Schicht 5 weitgehend reflektiert. Vorzugsweise reflektiert die Schicht 5 die von den biolumineszenten und/oder fluoreszenten Markern emittierten Photonen diffus, so daß ein Teil der reflektierten Strahlung in Richtung der CCD-Kameras 1, 2 reflektiert und dort detektiert wird. Das Gesichtsfeld 34 der CCD-Kameras ist in Figur 4 dargestellt.

Zur Untersuchung der Maus 8 genügt grundsätzlich auch nur die halbe in Figur 4 dargestellte Vorrichtung, also eine CCD-Kamera 1, eine Maske 23 und ein SPECT-Detektor 20 mit einer diffus reflektierenden Schicht 5. Die in Figur 4 gezeigte doppelte Anordnung hat jedoch den Vorteil eines insgesamt höheren Meßsignals, da die in beiden Richtungen emittierten Photonen detektiert werden und folglich eine höhere Auflösung der daraus berechneten Bilder.

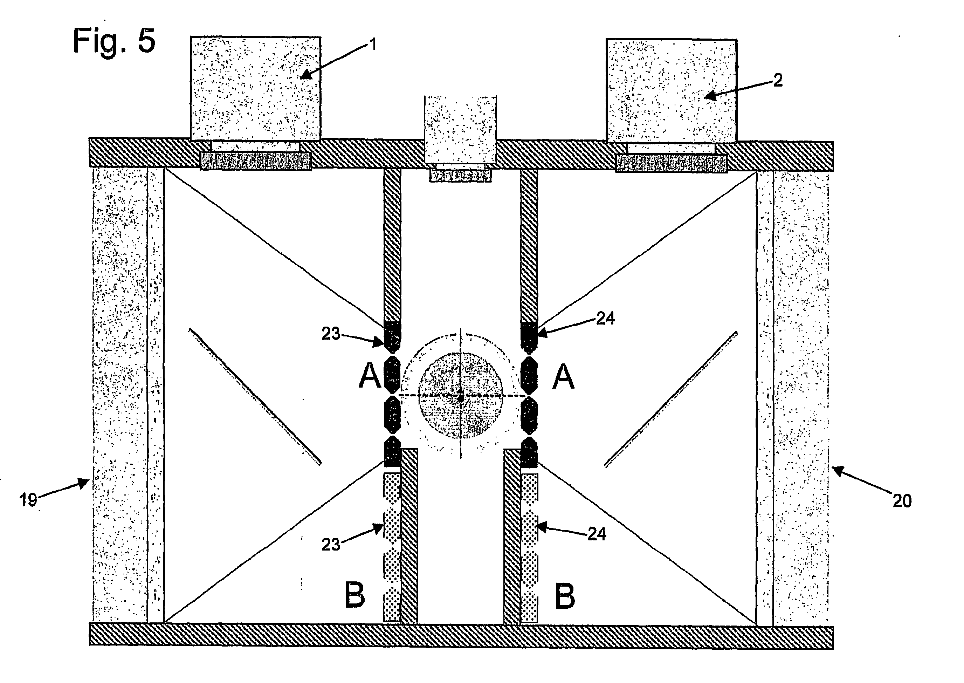

Figur 5 zeigt eine Abwandlung der in Figur 3 gezeigten Ausführungsform einer erfindungsgemäßen Vorrichtung mit verfahrbaren Masken.

Prinzipiell entspricht der Aufbau dieser Ausführungsform einer erfindungsgemäßen Vorrichtung dem in Figur 3 gezeigten. Zusätzlich sind bei dieser Abwandlung die erste und zweite Maske 23, 24 so befestigt, dass sie während der Akquisition aus den Gesichtsfeldern der CCD-Kameras 1, 2 (von Position A nach Position B) herausgeführt und wieder in die Anfangsposition (A) zurückgesetzt werden können. Befinden sich die Masken 23, 24 außerhalb der Gesichtsfelder der CCD-Kameras 1, 2 (Position B), so erhöht sich signifikant die Sensitivität dieser optischen bildgebenden Systeme. Die Detektion von radioistopischen Photonen ist in diesem Zustand (ohne Kollimation) jedoch nicht möglich. Daher sind die SPECT-Detektoren 19, 20 bei aus dem Strahlengang entfernten Masken 23, 24 inaktiv. Linsen werden in dieser Ausführungsform der vorliegenden Erfindung nicht benötigt. Die CCD-Kameras 1, 2 verfügen über eine Optik zur Fokussierung der von den bioluminiszenten und/oder fluoreszenten Markern emittierten Strahlen. Sind die Masken 23, 24 in der in Figur 5 gezeigten Ausführungsform der erfindungsgemäßen Vorrichtung in Position A, so werden bevorzugt die CCD-Kameras 1, 2 inaktiviert. Durch zeitlich alternierende Einführung der Masken 23, 24 in die Positionen A und B ergibt sich ein weiterer bevorzugter Anwendungsmodus des Designs aus Figur 3.

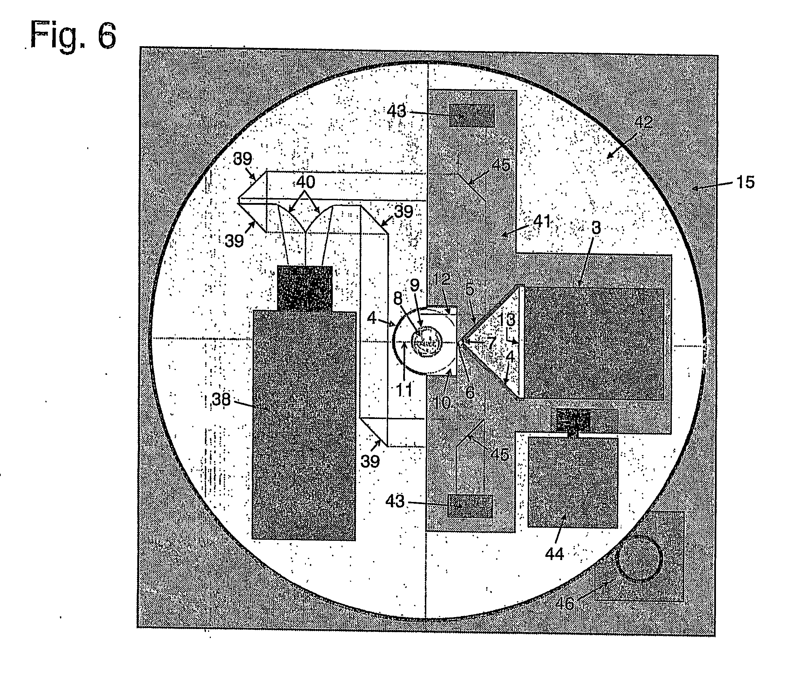

Der Figur 6 ist eine bevorzugte Abwandlung der in Figur 1 gezeigten Ausführungsform einer erfindungsgemäßen Vorrichtung mit nur einer CCD-Kamera zu entnehmen.

Bei dieser bevorzugten Ausführungsform der vorliegenden Erfindung wird das Prinzip der Bildgebung, wie in Figur 1 dargestellt, übernommen. Der Aufbau und die Wirkungsweise der SPECT-Kamera 3, einschließlich der Abschirmung 4, der Apertur 7 und der Anordnung und Wirkungsweise der reflektierenden Schicht 5 ist mit der dort beschriebenen Vorrichtung identisch. Abweichend werden jedoch gemäß dieser Ausführungsvariante die separaten Gesichtsfelder der reflektierenden Schicht 5 mittels einer Anordnung von Spiegeln 39, 40, 45 derart definiert, so dass diese im Objektiv der CCD-Kamera 38 und somit auf der lichtempfindlichen Matrix, aneinander angrenzend abgebildet werden. Die Spiegel 39 und 45 sind vorzugsweise planar beschaffen, während die Spiegel 40 konkav gewölbt ausgebildet werden können. Die Spiegel 45 sind einseitig durchlässig, so dass optional Laserstrahlen eingeführt werden können, um NIRF-Marker anzuregen.

Diese Art der Projektionszusammenfübrung kann gewählt werden, da die Ortsauflösung der CCD-Kamera 38 um mindestens eine Ordnung höher liegt als die physikalisch mögliche geometrische Auflösung im Objekt, hier der Maus 8, die aufgrund von Photonen-Streuungsprozessen im Objekt 8 begrenzt ist. Der Vorteil dieser Abwandlung ist eine räumlich kompaktbauendere und preiswertere Ausführungsform des Gegenstandes der vorliegenden Erfindung.

Um unterschiedlich große Tiere optimal abbilden zu können, ist in die in der Figur 6 dargestellten Ausführungsvariante die SPECT-Kamera 3 einschließlich der Abschirmung 4 der Apertur 7 und der reflektierenden Oberfläche 5 sowie Spiegel 45 und Laserstrahleinkopplungen 43, auf einer mittels eines Schrittmotors 44 verschiebbaren Bühne 41 montiert. Die Gesichtsfeldabbildung der CCD-Kamera 38 ist so definiert, dass die mittleren Abbildungslängen beider Halbfelder vom Objekt 8 zum Objektiv der CCD-Kamera 38 gleich groß sind. Diese Bedingung ist auch dann gewährleistet, wenn die radiale Position der verschiebbaren Bühne 41 zum Objekt 8 geändert wird. Die Bühne 41 einschließlich aller hierauf montierten Teilvorrichtungen, wie zum Beispiel der zum Antrieb erforderliche Schrittmotor 44, die Spiegel 39 und 40, die Abschirmung 4 sowie die CCD-Kamera 38 sind auf einer drehbaren Montageauflage 42 befestigt. Die Montageauflage 42 kann mittels eines Schrittmotors 46 um 360° rotiert werden, so dass die projektionsidentischen Gesichtsfelder beider Kamerasysteme zum Zwecke der Bilddatengewinnung in jedem beliebigen gemeinsamen Projektionswinkel zum Objekt 8 angeordnet werden können. Während die Montageauflage 42 am Gehäuse 15 drehbar aufgenommen ist, ist der Schrittmotor 46 fest mit dem Gehäuse 15 verbunden.

Bei allen beschriebenen Ausführungsformen der vorliegenden Erfindung ist bzw. sind die Aperturen 7 angesenkte längliche Öffnungen. Diese angesenkten Öffnungen sehen z.B. wie in der Ausschnittsvergrößerung A in Figur 4 aus. Die Aperturen verengen sich dabei von außen her 36 bis auf einen bestimmten Durchmesser 37, den sie anschließend bis zur Mitte beibehalten. Dadurch wird die Penetration von isotopischen Photonen im Bereich konisch sich verjüngender Aperturränder verringert.

### Bezugszeichenliste

- 1: erste CCD-Kamera
- 2: zweite CCD-Kamera
- 3: SPECT-Detektor
- 4: Abschirmung
- 5: Schicht
- 6: Biegekante der Schicht
- 7: Apertur
- 8: Maus
- 9: Röhre
- 10: erster Strahlenverlauf
- 11: zweiter Strahlenverlauf
- 12: dritter Strahlenverlauf
- 13: Szintillationskristallfeld
- 14: Photomultiplikatorfeld
- 15: Gehäuse
- 16: Subjekt
- 17: dritte CCD-Kamera
- 18: vierte CCD-Kamera
- 19: zweiter SPECT-Detektor
- 20: erster SPECT-Detektor
- 23: erste Maske
- 24: zweite Maske
- 25: erste Linse
- 26: zweite Linse
- 27: erster Reflektor
- 28: zweiter Reflektor
- 29: erster optischer Strahl
- 30: zweiter optischer Strahl
- 31: Akzeptanz-Kegel der SPECT-Detektoren
- 32: erste Kammer
- 33: zweite Kammer
- 34: Gesichtsfeld der CCD-Kameras
- 35: Positionssensor
- 36: Verengung der Apertur
- 37: konstanter Durchmesser in der Mitte der Öffnung
- 38: weitere CCD-Kamera
- 39: Spiegel, planar
- 40: Spiegel, konkav gewölbt
- 41: Bühne, radial verschiebbar
- 42: Montageauflage, drehbar
- 43: Laserstrahleinkopplung
- 44: 1. Schrittmotor
- 45: Spiegel, einseitig durchlässig
- 46: 2. Schrittmotor

## Patentansprüche

1. Bildgebendes Verfahren zur gleichzeitigen Bestimmung von in vivo Verteilungen biolumineszenter und/oder fluoreszenter Marker und radioaktiver Marker unter gleichen Projektionswinkeln, wobei die Verteilung der biolumineszenten und/oder fluoreszenten Marker durch separaten Nachweis von Photonen mit einer ersten mittleren Energie, die die biolumineszenten und/oder fluoreszenten Marker emittieren, mittels mindestens einem ersten Detektor bestimmt wird und die Verteilung der radioaktiven Marker durch gleichzeitigen separaten Nachweis von Photonen mit einer zweiten mittleren Energie, die die radioaktiven Marker emittieren, mittels mindestens einem zweiten Detektor bestimmt wird.

2. Bildgebendes Verfahren gemäß Anspruch 1, **dadurch gekennzeichnet, dass** die Photonen der biolumineszenten und/oder fluoreszenten Marker mit der ersten mittleren Energie und die Photonen der radioaktiven Marker mit der zweiten mittleren Energie zum separaten Nachweis mit Hilfe einer Schicht (5) getrennt werden, wobei die Schicht (5) die Photonen in Abhängigkeit von ihrer Energie im wesentlichen reflektiert oder transmittiert.

3. Bildgebendes Verfahren gemäß Anspruch 2, **dadurch gekennzeichnet, dass** die Schicht (5) dazu dient, die Photonen der biolumineszenten und/oder fluoreszenten Marker in Richtung des mindestens einen ersten Detektors zu reflektieren und die Photonen der radioaktiven Marker in Richtung des mindestens einen zweiten Detektors zu transmittieren.

4. Bildgebendes Verfahren gemäß einem der Ansprüche 1 bis 3, **dadurch gekennzeichnet, dass** die biolumineszenten und/oder fluoreszenten Marker mindestens einen Marker aus der Gruppe der Marker der Luziferase-Reporter, der Marker-Moleküle mit Emissionswellenlängen im Nahinfrarotbereich (NIRF-Moleküle) und der Moleküle des GFP (des grünfluoreszierenden Proteins) umfassen.

5. Bildgebendes Verfahren gemäß einem der Ansprüche 1 bis 4, **dadurch gekennzeichnet, dass** die radioaktiven Marker mindestens einen Marker aus der Gruppe As-72, Br-75, Co-55, Cu-61, Cu-64, Cu-67, Ga-67, Gd-153, 1-123, I-125, I-131, In-111, Ru-97, Tl-201, Tc-99m und Xe-133 umfassen.

6. Bildgebendes Verfahren gemäß einem der Ansprüche 1 bis 5, **dadurch gekennzeichnet, dass** der Nachweis der die erste mittlere Energie aufweisenden Photonen mit mindestens einer CCD-Kamera (1, 2; 38) und der Nachweis der die zweite mittlere Energie aufweisenden Photonen mit mindestens einem Einzelphotonenemissions-Computertomographie (SPECT)-Detektor (3), der einen Kollimator mit mindestens einer Apertur (7) umfasst, durchgeführt wird.

7. Bildgebendes Verfahren zur abwechselnden Bestimmung von in vivo Verteilungen biolumineszenter und/oder fluoreszenter Marker und von in vivo Verteilungen radioaktiver Marker mit einem gemeinsamen Messaufbau unter gleichen Projektionswinkeln, wobei die Verteilung der biolumineszenten und/oder fluoreszenten Marker durch separaten Nachweis von Photonen mit einer ersten mittleren Energie, die die biolumineszenten und/oder fluoreszenten Marker emittieren, mittels mindestens einem ersten Detektor bestimmt wird und abwechselnd dazu die Verteilung der radioaktiven Marker durch separaten Nachweis von Photonen mit einer zweiten mittleren Energie, die die radioaktiven Marker emittieren, mittels mindestens einem zweiten Detektor bestimmt wird.

8. Vorrichtung zur Durchführung des bildgebenden Verfahrens gemäß einem der Ansprüche 1 bis 7, enthaltend mindestens eine CCD-Kamera (1, 2, 38) als 1. Detektor, mindestens einen Einzelphotonenemissions-Computertomographie (SPECT)-Detektor (3) als zweiten Detektor und eine Schicht (5), die die Photonen der biolumineszenten und/oder fluoreszenten Marker im wesentlichen reflektiert und die Photonen der radioaktiven Marker im wesentlichen transmittiert.

9. Vorrichtung gemäß Anspruch 8, **dadurch gekennzeichnet, dass** der mindestens eine SPECT-Detektor (3) ein Szintillations-Kristallfeld (13) mit einer Vielzahl von Szintillations-Kristallen und ein ortsauflösendes Photomultiplikator-Feld (14) umfasst.

10. Vorrichtung gemäß einem der Ansprüche 8 oder 9, umfassend zwei einander zugewandte gekühlte CCD-Kameras (1, 2; 38), einen senkrecht zu den CCD-Kameras (1, 2, 38) angeordneten SPECT-Detektor (3), eine vor dem SPECT-Detektor (3) angeordnete, in einem 90°-Winkel gebogene, Abschirmung (4) und eine auf der Abschirmung (4) befestigte, ebenfalls in einem 90°-Winkel gebogene Schicht (5), deren Biegekante (6) auf der Biegekante der Abschirmung (4) liegt, wobei die Schicht (5) eine Apertur (7) in der Abschirmung (4) überdeckt und die von den biolumineszenten und/oder fluoreszenten Markern emittierten Photonen weitgehend reflektiert und die von den radioaktiven Markern emittierten Photonen weitgehend transmittiert.

11. Vorrichtung gemäß einem der Ansprüche 8 oder 9, umfassend zwei parallel und entgegengesetzt zueinander ausgerichtete gekühlte CCD-Kameras (1, 2; 38) zwischen zwei einander zugewandten SPECT-Detektoren (19, 20) und zwei Masken (23, 24) mit mindestens je zwei Aperturen (7), wobei sich je eine Schicht (5) vor den SPECT-Detektoren (19, 20) befindet, die die von den biolumineszenten und/oder fluoreszenten Markern emittierten Photonen weitgehend reflektiert und die von den radioaktiven Markern emittierten Photonen weitgehend transmittiert.

12. Vorrichtung gemäß Anspruch 11, **dadurch gekennzeichnet, dass** die Schicht (5) die von den biolumineszenten und/oder fluoreszenten Markern emittierten Photonen diffus reflektiert.

13. Vorrichtung gemäß einem der Ansprüche 8 oder 9, umfassend zwei in gleicher Richtung ausgerichtete und voneinander beabstandete gekühlte CCD-Kameras (1, 2; 38), zwei senkrecht zu den CCD-Kameras (1, 2; 38) angeordnete SPECT-Detektoren (19, 20), zwei Masken (23, 24) mit mindestens je zwei Aperturen (7) und zwei Linsen (25, 26) zwischen den zwei SPECT-Detektoren (19, 20), zwei im wesentlichen eine Schicht (5) umfassende Reflektoren (27, 28), die so ausgerichtet sind, dass sie die von den biolumineszenten und/oder fluoreszenten Markern emittierten, durch die Aperturen (7) in den Masken (23, 24) in Richtung SPECT-Detektoren (19, 20) durchgelassenen und mit den Linsen (25, 26) fokussierten Photonen weitgehend in Richtung der CCD-Kameras (1, 2) reflektieren.

14. Vorrichtung gemäß Anspruch 13, umfassend einen Positionssensor (35) zur Bestimmung der aktuellen Position eines zu untersuchenden Subjekts.

15. Vorrichtung gemäß einem der Ansprüche 13 oder 14, **dadurch gekennzeichnet, dass** die Masken (23, 24) während einer Messung aus den Gesichtsfeldern der CCD-Kameras (1, 2; 38) herausfahrbar (Position B) und in die Gesichtsfelder hereinfahrbar (Position A) sind.

16. Vorrichtung gemäß einem der Ansprüche 10 bis 15, **dadurch gekennzeichnet, dass** die Apertur (7) eine angesenkte längliche Öffnung ist.

17. Vorrichtung gemäß einem der Ansprüche 8 oder 9, umfassend eine gekühlte CCD-Kamera (38), einen senkrecht zu der CCD-Kamera (38) angeordneten SPECT-Detektor (3), eine vor dem SPECT-Detektor (3) angeordnete, in einem 90°-Winkel abgebogene Abschirmung (4) und eine auf der Abschirmung (4) befestigte, ebenfalls in einem 90°-Winkel gebogene reflektierende Schicht (5), deren Biegekante (6) auf der Biegekante der Abschirmung (4) liegt, wobei die reflektierende Schicht (5) eine Apertur (7) in der Abschirmung (4) überdeckt, wobei der SPECT-Detektor (3) die Abschirmung (4) samt reflektierender Schicht (5) und Apertur (7) sowie Spiegel (45) und Lasereinkopplungen (43) auf einer verschiebbar ausgebildeten Bühne (41) aufgenommen sind.

18. Vorrichtung gemäß Anspruch 17, **dadurch gekennzeichnet, dass** die verschiebbare Bühne (41) auf einer drehbar ausgebildeten Montageauflage (42) angeordnet ist.

19. Vorrichtung gemäß Anspruch 17, **dadurch gekennzeichnet, dass** separate Gesichtsfelder der reflektierenden Schicht (5) mittels einer Anordnung von Spiegeln (39, 40, 45) im Objektiv der CCD-Kamera (38) aneinander angrenzend abgebildet werden.

20. Vorrichtung gemäß Anspruch 17, **dadurch gekennzeichnet, dass** an der verschiebbaren Bühne (41) Laserstrahleinkopplungen (43) sowie einseitig durchlässige Spiegel (45) einander gegenüberliegend aufgenommen sind, um im Objekt (8) NIRF-Marker mittels Laserstrahlen anzuregen.

21. Verwendung eines bildgebenden Verfahrens gemäß einem der Ansprüche 1 bis 7 für in vivo Studien an kleinen Tieren, zur in vivo Beobachtung von Gen-Expression und zur Brust-, Prostata-, Hauttumoren- und Schilddrüsenbildgebung verwendet.

## Claims

1. Imaging method for simultaneously determining in vivo distributions of bioluminescent and/or fluorescent markers and radioactive markers at identical projection angles, the distribution of the bioluminescent and/or fluorescent markers being determined by separate detection of photons having a first average energy, which are emitted by the bioluminescent and/or fluorescent markers, by means of at least one first detector and the distribution of the radioactive markers being determined by simultaneous separate detection of photons having a second average energy, which are emitted by the radioactive markers, by means of at least one second detector.

2. Imaging method according to Claim 1, **characterized in that** the photons of the bioluminescent and/or fluorescent markers having the first average energy and the photons of the radioactive markers having the second average energy are separated for the separate detection with the aid of a layer (5), the layer (5) essentially reflecting or transmitting the photons in a manner dependent on their energy.

3. Imaging method according to Claim 2, **characterized in that** the layer (5) serves for reflecting the photons of the bioluminescent and/or fluorescent markers in the direction of the at least one first detector and for transmitting the photons of the radioactive markers in the direction of the at least one second detector.

4. Imaging method according to one of Claims 1 to 3, **characterized in that** the bioluminescent and/or fluorescent markers comprise at least one marker from the group consisting of the markers of the luciferase reporters, the marker molecules having emission wavelengths in the near infrared range (NIRF molecules) and the molecules of the GFP (green fluorescent protein).

5. Imaging method according to one of Claims 1 to 4, **characterized in that** the radioactive markers comprise at least one marker from the group As-72, Br-75, Co-55, Cu-61, Cu-67, Ga-67, Gd-153, 1-123, 1-125, 1-131, In-111, Ru-97, Tl-201, Tc-99m and Xe-133.

6. Imaging method according to one of Claims 1 to 5, **characterized in that** the detection of the photons having the first average energy is carried out by means of at least one CCD camera (1, 2; 38) and the detection of the photons having the second average energy is carried out by means of at least one single photon emission computer tomography (SPECT) detector (3) comprising a collimator with at least one aperture (7).

7. Imaging method for alternately determining in vivo distributions of bioluminescent and/or fluorescent markers and in vivo distributions of radioactive markers by means of a common measurement setup at identical projection angles, the distribution of the bioluminescent and/or fluorescent markers being determined by separate detection of photons having a first average energy, which are emitted by the bioluminescent and/or fluorescent markers, by means of at least one first detector and, alternately with respect thereto, the distribution of the radioactive markers being determined by separate detection of photons having a second average energy, which are emitted by the radioactive markers, by means of at least one second detector.

8. Apparatus for carrying out the imaging method according to one of Claims 1 to 7, containing at least one CCD camera (1, 2, 38) at 1st detector, at least one single photon emission computer tomography (SPECT) detector (3) as second detector and a layer (5), which essentially reflects the photons of the bioluminescent and/or fluorescent markers and essentially transmits the photons of the radioactive markers.

9. Apparatus according to Claim 8, **characterized in that** the at least one SPECT detector (3) comprises a scintillation crystal array (13) with a multiplicity of scintillation crystals and a spatially resolving photomultiplier array (14).

10. Apparatus according to either of Claims 8 and 9, comprising two cooled CCD cameras (1, 2; 38) facing one another, a SPECT detector (3) arranged perpendicular to the CCD cameras (1, 2, 38), a shielding (4) arranged in front of the SPECT detector (3) and bent at an angle of 90°, and a layer (5) fixed on the shielding (4) and likewise bent at an angle of 90°, the bending edge (6) of said layer lying on the bending edge of the shielding (4), the layer (5) covering an aperture (7) in the shielding (4) and largely reflecting the photons emitted by the bioluminescent and/or fluorescent markers and largely transmitting the photons emitted by the radioactive markers.

11. Apparatus according to either of Claims 8 and 9, comprising two cooled CCD cameras (1, 2; 38) oriented parallel and oppositely to one another between two SPECT detectors (19, 20) facing one another and two masks (23, 24) with at least two apertures (7) in each case, a respective layer (5) being situated in front of the SPECT detectors (19, 20), said layer largely reflecting the photons emitted by the bioluminescent and/or fluorescent markers and largely transmitting the photons emitted by the radioactive markers.

12. Apparatus according to Claim 11, **characterized in that** the layer (5) diffusely reflects the photons emitted by the bioluminescent and/or fluorescent markers.

13. Apparatus according to either of Claims 8 and 9, comprising two cooled CCD cameras (1, 2; 38) oriented in the same direction and spaced apart from one another, two SPECT detectors (19, 20) arranged perpendicular to the CCD cameras (1, 2; 38), two masks (23, 24) with at least two apertures (7) in each case and two lenses (25, 26) between the two SPECT detectors (19, 20), two reflectors (27, 28) essentially comprising a layer (5), which are oriented in such a way that they largely reflect, in the direction of the CCD cameras (1, 2), the photons that are emitted by the bioluminescent and/or fluorescent markers, transmitted through the apertures (7) in the masks (23, 24) in the direction of the SPECT detectors (19, 20) and focused by the lenses (25, 26).

14. Apparatus according to Claim 13, comprising a position sensor (35) for determining the current position of a subject to be examined.

15. Apparatus according to either of Claims 13 and 14, **characterized in that** the masks (23, 24), during a measurement, can be moved out of the fields of view of the CCD cameras (1, 2; 38) (position B) and can be moved into the fields of view (position A).

16. Apparatus according to one of Claims 10 to 15, **characterized in that** the aperture (7) is a countersunk elongate opening.

17. Apparatus according to either of Claims 8 and 9, comprising a cooled CCD camera (38), a SPECT detector (3) arranged perpendicular to the CCD camera (38), a shielding (4) arranged in front of the SPECT detector (3) and bent away at an angle of 90°, and a reflective layer (5) fixed on the shielding (4) and likewise bent at an angle of 90°, the bending edge (6) of said layer lying on the bending edge of the shielding (4), the reflective layer (5) covering an aperture (7) in the shielding (4), the SPECT detector (3), the shielding (4) together with reflective layer (5) and aperture (7) and also mirrors (45) and laser coupling-in arrangements (43) being accommodated on a platform (41) formed in displaceable fashion.

18. Apparatus according to Claim 17, **characterized in that** the displaceable platform (41) is arranged on a mounting support (42) formed in rotatable fashion.

19. Apparatus according to Claim 17, **characterized in that** separate fields of view of the reflective layer (5) are imaged in a manner adjoining one another by means of an arrangement of mirrors (39, 40, 45) in the objective of the CCD camera (38).

20. Apparatus according to Claim 17, **characterized in that** laser beam coupling-in arrangements (43) and also mirrors (45) that are transmissive on one side are accommodated opposite one another on the displaceable platform (41) in order to excite NIRF markers in the object (8) by means of laser beams.

21. Use of an imaging method according to one of Claims 1 to 7 for in vivo studies on small animals, for in vivo observation of gene expression and for breast, prostate, skin tumor and thyroid gland imaging.

## Revendications

1. Procédé d'imagerie permettant de déterminer simultanément les répartitions *in vivo* de marqueurs bioluminescents et/ou fluorescents et de marqueurs radioactifs sous des angles de projection identiques, la répartition des marqueurs bioluminescents et/ou fluorescents étant déterminée par la détection séparée des photons présentant une première énergie intermédiaire émise par les marqueurs bioluminescents et/ou fluorescents au moyen d'au moins un premier détecteur, et la répartition des marqueurs radioactifs étant déterminée par la détection séparée simultanée des photons présentant une deuxième énergie intermédiaire émise par les marqueurs radioactifs au moyen d'au moins un deuxième détecteur.

2. Procédé d'imagerie selon la revendication 1, **caractérisé en ce que** les photons des marqueurs bioluminescents et/ou fluorescents présentant la première énergie intermédiaire et les photons des marqueurs radioactifs présentant la deuxième énergie intermédiaire sont séparés en vue de permettre la détection séparée à l'aide d'une couche (5), laquelle couche (5) reflète ou transmet principalement les photons en fonction de leur énergie.

3. Procédé d'imagerie selon la revendication 2, **caractérisé en ce que** la couche (5) sert à refléter les photons des marqueurs bioluminescents et/ou fluorescents dans la direction du au moins un premier détecteur et les photons des marqueurs radioactifs dans la direction du au moins un deuxième détecteur à des fins de transmission.

4. Procédé d'imagerie selon l'une quelconque des revendications 1 à 3, **caractérisé en ce que** les marqueurs bioluminescents et/ou fluorescents comprennent un marqueur appartenant au groupe des marqueurs du rapporteur luciférase, des molécules de marqueurs avec des longueurs d'onde d'émission s'inscrivant dans la plage proche de l'infrarouge (molécule NIRF) et des molécules de PVF (la protéine à fluorescence verte).

5. Procédé d'imagerie selon l'une quelconque des revendications 1 à 4, **caractérisé en ce que** les marqueurs radioactifs comprennent au moins un marqueur appartenant au groupe As-72, Br-75, Co-55, Cu-61, Cu-64, Cu-67, Ga-67, Gd-153, I-123, I-125, I-131, In-111, Ru-97, Tl-201, Tc-99m et Xe-133.

6. Procédé d'imagerie selon l'une quelconque des revendications 1 à 5, **caractérisé en ce que** la détection des photons présentant la première énergie intermédiaire est réalisée au moyen d'au moins une caméra CCD (1, 2; 38) et la détection des photons présentant la deuxième énergie intermédiaire est réalisée au moyen d'au moins un détecteur (TEPU/SPECT) (3) de tomographie à émission de photons uniques assisté par ordinateur, comprenant un collimateur avec au moins une ouverture (7).

7. Procédé d'imagerie permettant de déterminer de façon alternée des répartitions *in vivo* de marqueurs bioluminescents et/ou fluorescents et des répartitions *in vivo* de marqueurs radioactifs à l'aide d'un système de mesure commun sous des angles de projection identiques, la répartition des marqueurs bioluminescents et/ou fluorescents étant déterminée par la détection séparée des photons présentant une première énergie intermédiaire émise par les marqueurs bioluminescents et/ou fluorescents au moyen d'au moins un premier détecteur, et la répartition des marqueurs radioactifs étant déterminée de façon alternée par la détection séparée simultanée des photons présentant une deuxième énergie intermédiaire émise par les marqueurs radioactifs au moyen d'au moins un deuxième détecteur.

8. Dispositif permettant la mise en oeuvre du procédé d'imagerie selon l'une quelconque des revendications 1 à 7, comprenant au moins une caméra CCD (1, 2; 38) utilisée comme premier détecteur, au moins un détecteur (TEPU/SPECT) (3) de tomographie à émission de photons uniques assisté par ordinateur, utilisé comme deuxième détecteur et une couche (5) qui reflète essentiellement les photons des marqueurs bioluminescents et/ou fluorescents et qui transmet essentiellement les photons des marqueurs radioactifs.

9. Dispositif selon la revendication 8, **caractérisé en ce que** le au moins un détecteur TEPU/SPECT (3) comprend un champ cristallin de scintillation (13) comprenant une multitude de cristaux de scintillations et un champ multiplicateur (14) de photons à résolution localisée.

10. Dispositif selon l'une quelconque des revendications 8 ou 9, comprenant deux caméras CCD (1, 2; 38) refroidies tournées l'une vers l'autre, un détecteur TEPU/SPECT (3) agencé perpendiculairement aux caméras CCD (1, 2; 38), un blindage (4) courbé selon un angle de 90° agencé devant le détecteur TEPU/SPECT (3) et une couche (5), également pliée selon un angle de 90°, fixée sur le blindage (4) dont l'arête de pliage (6) repose sur l'arête de pliage du blindage (4), la couche (5) recouvrant une ouverture (7) située dans le blindage (4) et reflétant massivement les photons émis par les marqueurs bioluminescents et/ou fluorescents et transmettant massivement les photons émis par les marqueurs radioactifs.

11. Dispositif selon l'une quelconque des revendications 8 ou 9, comprenant deux caméras CCD (1, 2; 38) refroidies alignées l'une face à l'autre de façon opposée et parallèle, agencées entre deux détecteurs TEPU/SPECT (19, 20) tournés l'un vers l'autre, et deux masques (23, 24) présentant chacun au moins deux ouvertures (7), une couche (5) se trouvant à chaque fois devant les détecteurs TEPU/SPECT (19, 20), laquelle couche reflète massivement les photons émis par les marqueurs bioluminescents et/ou fluorescents et transmet massivement les photons émis par les marqueurs radioactifs.

12. Dispositif selon la revendication 11, **caractérisé en ce que** la couche (5) reflète de façon diffuse les photons émis par les marqueurs bioluminescents et/ou fluorescents.

13. Dispositif selon l'une quelconque des revendications 8 ou 9, comprenant deux caméras CCD (1, 2; 38) refroidies distantes l'une de l'autre et alignées dans la même direction et deux détecteurs TEPU/SPECT (19, 20) agencés perpendiculairement aux caméras CCD (1, 2; 38), deux masques (23, 24) présentant chacun au moins deux ouvertures (7) et deux lentilles (25, 26) entre les deux détecteurs TEPU/SPECT (19, 20), deux réflecteurs (27, 28) comprenant principalement une couche (5), qui sont alignés de façon à refléter les photons, émis par les marqueurs bioluminescents et/ou fluorescents, traversant l'ouverture (7) située dans les masques (23, 24) en direction des détecteurs TEPU/SPECT (19, 20) et focalisés à l'aide des lentilles (25, 26) dans la direction des caméras CCD (1, 2).

14. Dispositif selon la revendication 13, comprenant un capteur de position (35) permettant de déterminer la position actuelle d'un sujet à examiner.

15. Dispositif selon l'une quelconque des revendications 13 ou 14, **caractérisé en ce que** les masques (23, 24) peuvent être exclus durant une mesure des champs de vision des caméras CCD (1, 2; 38) (position B) et ramenés dans les champs de vision (position A).

16. Dispositif selon l'une quelconque des revendications 10 à 15, **caractérisé en ce que** l'ouverture (7) est une ouverture longitudinale creusée.

17. Dispositif selon l'une quelconque des revendications 8 ou 9, comprenant une caméra CCD refroidie (38), un détecteur (3) TEPU/SPECT agencé perpendiculairement à la caméra CCD (38), un blindage (4) plié selon un angle de 90° agencé devant le détecteur TEPU/SPECT (3) et une couche réfléchissante (5), également pliée selon un angle de 90°, fixée sur le blindage (4) dont l'arête de pliage (6) repose sur l'arête de pliage du blindage (4), la couche réfléchissante (5) recouvrant une ouverture (7) dans le blindage (4), le détecteur TEPU/SPECT (3), le blindage (4) ainsi que la couche réfléchissante (5), l'ouverture (7) ainsi que le miroir (45) et les couplages laser (43) étant tous sans exception placés sur un plan (41) formé de façon déplaçable.

18. Dispositif selon la revendication 17, **caractérisé en ce que** le plan déplaçable (41) est agencé sur un support de montage formé de façon à pouvoir pivoter (42).

19. Dispositif selon la revendication 17, **caractérisé en ce que** des champs de vision distincts de la couche réfléchissante (5) sont représentés de façon adjacente l'un par rapport à l'autre au moyen d'un agencement de miroirs (39, 40, 45) dans l'objectif de la caméra CCD (38).

20. Dispositif selon la revendication 17, **caractérisé en ce qu'**au niveau du plan déplaçable (41), les couplages de faisceau laser (43) ainsi qu'un miroir (45) transparent sur une face sont placés en vis-à-vis pour exciter les marqueurs NIRF dans l'objet (8) au moyen de faisceaux laser.

21. Utilisation d'un procédé d'imagerie selon l'une quelconque des revendications 1 à 7 pour des études *in vivo* chez de petits animaux, destinées à l'observation *in vivo* de l'expression de gène et pour l'imagerie de tumeurs cutanées, du sein, de la prostate et de la glande thyroïde.
